(19) Europäisches Patentamt

European Patent Office

Office européen des brevets

(11) **EP 2 364 674 A1**

(12) **EUROPEAN PATENT APPLICATION**

(43) Date of publication:
**14.09.2011 Bulletin 2011/37**

(51) Int Cl.:
*A61F 2/42* (2006.01)     *A61F 2/00* (2006.01)

(21) Application number: **10002528.7**

(22) Date of filing: **10.03.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(71) Applicant: **Aequos Endoprothetik Gmbh
82166 Gräfelfing (DE)**

(72) Inventor: **The designation of the inventor has not yet been filed**

(74) Representative: **Leidescher, Thomas
Zimmermann & Partner
Postfach 330 920
80069 München (DE)**

(54) **Saddle-shaped mechanical joint**

(57) A mechanical joint, in particular an endoprosthesis, e.g. for a carpometacarpal joint, is provided. The mechanical joint includes a first part of the joint and a second part of the joint. The first part of the joint includes a first curved surface, and the second part of the joint includes a second curved surface. The first and second parts are adapted to form, in a first position of the mechanical joint, an articulated connection in a contact point under compressive force. The first and second curved surfaces are saddle-shaped at least in a region including the contact point.

Fig. 1

Printed by Jouve, 75001 PARIS (FR)

## Description

### FIELD OF THE INVENTION

The embodiments of the present invention relate to a mechanical joint. Typically, the mechanical joint can be an endoprosthesis. For example, the mechanical joint may be an endoprosthesis replacing a human joint such as the carpometacarpal joint of the thumb. Particular embodiments of the present invention relate to a mechanical joint for handicapping or restricting a relative axial rotation of a first and second part of the joint that are articulated by a compressive force.

### BACKGROUND OF THE INVENTION

Osteoarthritis (OA) of the carpometacarpal joint of the thumb (CMCJ) is a frequent clinical problem. This problem is present in radiographs with a prevalence of about 33% in postmenopausal women. This large prevalence of osteoarthritis (OA) is attributed to the high contact pressures exposing the cartilage of the CMCJ.

The CMCJ may be replaced by an artificial, mechanical joint when the natural joint is not functional anymore or the pain of OA becomes unbearable.

Artificial carpometacarpal joints of the thumb in the form of an endoprosthesis are known, wherein the surfaces of a proximal and distal part of the joint are a convex oval and a concave oval, respectively. The distal, concave oval has a smaller curvature than the proximal, convex oval, and contacts the convex oval at one contact point under a compressive force exerted by muscles and/or ligaments.

The compressive force at the contact point is perpendicular to the surfaces of the proximal and distal parts of the joint, i.e. coincides with the direction of the normal to these surfaces. A relative rotation of the proximal and distal parts around an axis of rotation defined by the normal to the surfaces, respectively the direction of the compressive force, at the contact point will be called an axial rotation.

For the artificial joint with the proximal, convex oval part contacting the distal, concave oval part, the axial rotation is not hindered by the compressive force. However, such an axial rotation is unnatural at least for larger rotation angles, and is detrimental to the muscles and the ligaments.

Thus, to avoid this undesired effect, there is a need for an improved mechanical joint, in particular an improved mechanical joint for a thumb.

### SUMMARY OF THE INVENTION

In light of the above, a mechanical joint according to independent claim 1 is provided.

According to an embodiment, a mechanical joint, e.g. an endoprosthesis, for example an endoprosthesis for a carpometacarpal joint, is provided. The mechanical joint includes a first part of the joint and a second part of the joint. The first part of the joint includes a first curved surface, and the second part of the joint includes a second curved surface. The first and second parts are adapted to form, in a first position of the mechanical joint, an articulated connection in a contact point under a compressive force. The first and second curved surfaces are saddle-shaped at least in a region including the contact point.

The invention is also directed to methods by which the described mechanical joint operates and to uses thereof. It includes method steps for carrying out every function of the mechanical joint. The invention is also directed to a process of manufacturing the described mechanical joint.

### BRIEF DESCRIPTION OF THE DRAWINGS

So that the manner in which the above recited features of the present invention can be understood in detail, a more particular description of the invention, briefly summarized above, may be had by reference to embodiments. The accompanying drawings relate to embodiments of the invention and are described in the following:

Fig. 1 illustrates properties of saddle-shaped surfaces, which are used in embodiments of mechanical joints described herein;

Fig. 2a) shows a sectional view of a mechanical joint according to embodiments described herein;

Fig. 2b) shows a another sectional view of a mechanical joint according to embodiments described herein;

Fig. 2c) illustrates, in a top view, further properties of a mechanical joint according to some embodiments described herein;

Fig. 3a) illustrates a method of moving a mechanical joint according to embodiments described herein; and

Fig. 3b) illustrates a method of moving a mechanical joint according to embodiments described herein.

### DETAILED DESCRIPTION OF THE INVENTION

Reference will now be made in detail to the various embodiments of the invention, one or more examples of which are illustrated in the figures. Within the following description of the drawings, the same reference numbers refer to same components. Generally, only the differences with respect to individual embodiments are described. Each example is provided by way of explanation of the invention and is not meant as a limitation of the invention. For example, features illustrated or described as part of one embodiment can be used on or in conjunction with other embodiments to yield yet a further embodiment. It is intended that the present invention includes such modifications and variations.

According to embodiments described herein, a mechanical joint is provided. Without limiting the present invention, the mechanical joint will be described as a mechanical joint for a thumb, in particular a carpometacarpal joint (CMCJ), also called *articulatio carpometacarpale,* e.g. for a human thumb. However, the mechanical joint could also be an artificial joint for an animal thumb or for different human or animal joint. The mechanical joint typically is an endoprosthesis.

The mechanical joint includes a first part of the joint and a second part of the joint. The first part of the joint may e.g. be the proximal part of the CMCJ, i.e. an artificial *os trapezium.* The second part of the joint may e.g. be the distal of the CMCJ, i.e. an artificial *os metacarpale.* The roles might also be reversed. Without limitation, the first part will often be called the proximal part in the following, and the second part will often be called the distal part.

The first part of the joint includes a first curved surface, and the second part of the joint includes a second curved surface. The first and second parts are adapted to form, in a first position of the mechanical joint, an articulated connection in a contact point under a compressive force. The first position may be the neutral position of the joint. As opposed to the known mechanical joints, the mechanical joint of the present disclosure can handicap or restrict the axial rotation of the first and second parts relative to each other. In particular, the relative axial rotation may be allowed for small rotation angles, but the joint can provide a counter-torque that restricts the relative axial rotation beyond a certain angle of maximal substantially unhindered rotation. The counter-torque may solely be provided by a compressive force in conjunction with a specific shape of the curved surfaces of the first and second parts, as described below. The compressive force may be an axial compressive force, i.e. a force pulling the first and second parts together in the direction normal to the tangent plane of the contact point. The compressive force may be exerted by muscles and/or ligaments. In some embodiments, the mechanical joint is a joint for handicapping or restricting the relative axial rotation of the first and second part of the joint that are articulated by the compressive force.

The first and second curved surfaces are each saddle-shaped at least in a region including the contact point. The first and second curved surfaces may each be saddle-shaped. A saddle-shaped surface or region of a surface is a two-dimensional curved surface. A two-dimensional curved surface can be described by notions of differential geometry. The terminology of this field will be used. A saddle-shaped surface may include one or more saddle points. For a smooth function (whose graph is differentiable manifold), a saddle point is a stationary point such that the manifold in the neighborhood of that point is not entirely on any side of the tangent space at that point.

The saddle shape of the surface or surface region may be a local property in the neighbourhood of the contact point, or a global property. Typically, the contact point in the first position is a main saddle point of the saddle-shaped regions of the first and second curved surfaces. The saddle-shaped region of the first and/or the second curved surface may be a hyperbolic paraboloid (second order saddle surface) or, more typically, a saddle surface of higher order, e.g. third, fourth, or fifth order.

Any point on the two-dimensional curved surface has two principal radii of curvature with associated centers of curvature. The saddle point where the principal curvatures are extreme is called main saddle point. At any point, the principal directions of curvature that correspond to the principal radii of curvature are perpendicular to one another. In other words, the surface normal planes at the point and in the principal directions are perpendicular to one another, and both are perpendicular to the surface tangent plane at the point. At the main saddle point one of the principal radii of curvature is positive, the other negative.

At a saddle point, two planes including the saddle point exist that intersect the saddle-shaped surface at least locally in a straight line. These planes are surface normal planes, i.e. planes including the normal to the tangent plane in the saddle point. For each point on these straight lines the curvature in a direction along the line is zero.

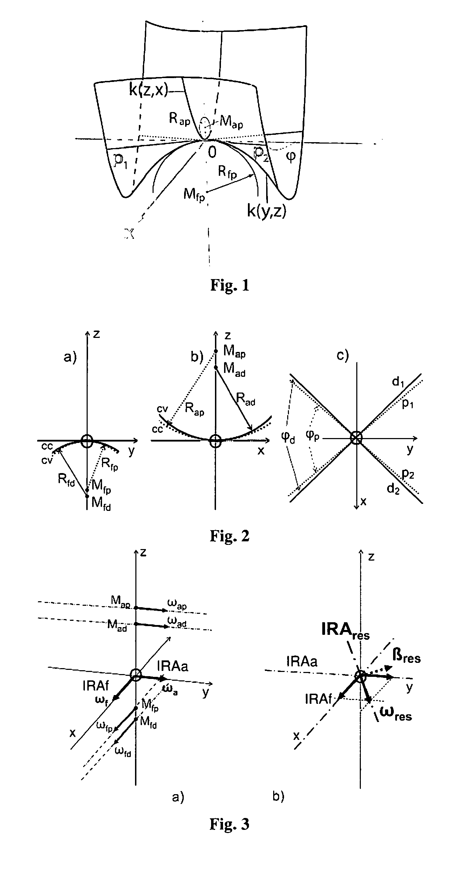

Fig. 1 illustrates properties of saddle surfaces. In Fig. 1, the proximal articulating surface in the artificial CMCJ is schematically and exemplarily shown. At the saddle point the surface can be sectioned by two planes which are perpendicular to each other so that the cutting edge of the planes coincides with the normal in the saddle point, and one line of intersection ($k(z,x)$) is extremely concave and the other ($k(y,z)$) extremely convex. The curvature radii at the saddle point along these curves represent the principal radii. At all other points of the saddle the respective principal curvatures would be smaller.

For the geometrical description the saddle point O is taken as the origin of the coordinate system, and the normal in O as the z-axis. The z-axis points from the proximal to the distal part of the artificial CMCJ. The directions of the principal curvatures are chosen as y- and x-axis, as shown in Fig. 1. Other planes which run through O and the z-axis produce lines of intersection whose curvatures in O are between the curvatures of the curves k $(z,x)$ and $k(y,z)$. Since the one curve is extreme convex and the other extreme concave there exist two straight lines (curvature=0) among the intersection lines p1 and p2. These straight lines at the saddle point O can be located by applying a straightedge to the saddle surface. The angle φ between both straight lines represents a possible geometric quantity to characterize a saddle surface.

As shown in Fig. 1, the x-z-plane is called the plane of ab-/adduction, and the y-z-plane is called the plane of flexion/extension. The following notation is used: Curve k(z,x) is a concave contour in the plane of ab-/adduction; $R_{ap}$ is a respective radius of curvature at the saddle point O; $M_{ap}$ is a corresponding center of curvature. Curve k(y,z) is a convex contour in the plane of flexion/extension; $R_{fp}$ is a respective radius of curvature at the saddle point O; $M_{fp}$ is a corresponding center of curvature. Lines p1, p2 are the respective straight lines of the saddle surface through the saddle point O.

Likewise, as shown in Figs. 2a)-2c), the distal articulating surface in the CMCJ has a saddle-shaped region around the contact point, and the following notation is used: $R_{ad}$ is radius of curvature of the distal surface at the saddle point O; $M_{ad}$ is a corresponding center of curvature. $R_{fd}$ is a radius of curvature of the distal surface at the saddle point O; $M_{fd}$ is a corresponding center of curvature. Lines d1, d2 are the respective straight lines of the distal saddle surface through the saddle point O. Therein, the saddle point O is the contact point of the proximal and distal surfaces. In Figs. 2a)-2c), full lines represent contours of the distal articulating surface, and dotted lines represent contours of the proximal articulating surface.

Fig. 2a) shows an intersection through the CMCJ in y-z-plane, i.e. the plane of flexion/extension. In flexion/extension, the convexly shaped surface of the artificial *os trapezium* (proximal part) articulates with the concavely shaped surface of the artificial *os metacarpale* (distal part). In saddle point O, the contour of the proximal surface is stronger curved. Hence, the center of curvature $M_{fp}$ of the proximal contour lies more distal (farther in positive z-direction) than the center of curvature $M_{fd}$ of the distal surface. The proximal and distal parts of the mechanical joint are incongruent in the plane of flexion/extension, i.e. their respective surfaces have incongruent curvature at the point of contact O.

Fig. 2b) shows an intersection through the CMCJ in x-z-plane, i.e. the plane of ab-/adduction. In ab/adduction the concavely shaped surface of the artificial *os trapezium* (proximal part) articulates with the convexly shaped surface of the artificial *os metacarpale I* (distal part). In saddle point O, the contour of the distal surface is stronger curved. Hence, the center of curvature $M_{ap}$ of the proximal contour lies more distal (farther in positive z-direction) than the center of curvature $M_{ad}$ of the distal surface. The proximal and distal parts of the mechanical joint are incongruent in the plane of ab-/adduction, i.e. their respective surfaces have incongruent curvature at the point of contact O.

Generally, according to some embodiments described herein, the first and second curved surfaces are incongruent at the contact point in one or both principal directions of curvature. Incongruence means that the absolute value of the principal radii in the respective direction(s) differ from each other. The first and second curved surfaces may be incongruent in their respective saddle-shaped regions in one or both principal directions of curvature. This means that incongruence holds for every point in the saddle-shaped region of the first curved surface assuming that it was a contact point to any point in the saddle-shaped region of the second curved surface.

According to some embodiments, the principal radii of curvature of the saddle-shaped region of the first curved surface at the contact point are $R_{ap}$ and $R_{fp}$, and the principal radii of curvature of the saddle-shaped region of the second curved surface at the contact point are $R_{ad}$ and $R_{fd}$. In these embodiments, at least one of the following relations may hold: $|R_{ap}| \neq |R_{ad}|$ and $|R_{fp}| \neq |R_{fd}|$, wherein $|\bullet|$ denotes the absolute value. In particular, e.g. as shown in Figs. 2a) and 2b) respectively, at least one of the following relations may hold: $|R_{ap}| > |R_{ad}|$ and $|R_{fp}| < |R_{fd}|$.

Figs. 2a)-2c) illustrate contours of intersections through the CMCJ in neutral position. The neutral position is the position in which the joint is not twisted, i.e. the relative axial rotation is zero. In Fig. 2c), the straight lines lie in the x-y-plane, which is the section plane through the straight lines of the proximal surface ($p_1$, $p_2$) and the distal surface ($d_1$, $d_2$) running through the respective contact point. In Figs. 2a)-2c), this contact point is the main saddle point. The position of the straight lines $p_1$, $p_2$, $d_1$, and $d_2$ is such that the two saddle-shaped regions of the proximal and distal parts are in a special neutral position. This neutral position is here characterized in that the angle between $d_1$ and $p_1$ is the same as the angle between $d_2$ and $p_2$. This special neutral position shall be named symmetric neutral position. In other embodiments, the mechanical joint may have a pre-tilt. That means, the saddle-shaped regions of the two parts of the joint may be arranged such that the neutral position has a certain bias or tilt into one direction of axial rotation. The mechanical joint could have a neutral position with a pre-tilt, e.g. with a pre-tilt from 0° to $\pm| \phi_p - \phi_d |/2$. For a pre-tilt of $\pm| \phi_p - \phi_d |/2$, the pair of straight lines $p_1$ and $d_1$, or the pair of straight lines $p_2$ and $d_2$ would coincide. In this case, first and second parts of the mechanical joint form, in the neutral position of the mechanical joint, an articulated connection under the compressive force in a contact line of the first and second curved surfaces. This case shall be included when specifying that first and second parts form a connection in a contact point.

According to some embodiments, as illustrated in Fig. 2c), $p_1$ and $p_2$ form an angle $\phi_p$ in a first sector, and $d_1$ and $d_2$ form an angle $\phi_d$ in a second sector. The first and second sectors are corresponding sectors, i.e. sectors with maximal overlap. A maximal overlap is e.g. given for sectors overlapping by more than 45°. For example, in Fig. 2c), the first and second sectors are the sectors in which the angles $\phi_p$ and $\phi_d$ are graphically illustrated. They overlap in an angle equal to $\phi_p$, which is larger than 45°. In Fig. 2c), the angles $\phi_p$ and $\phi_d$ are measured in the sector including the y-direction (direction of ab-/adduction). Generally, one, two, or all three of the following relations may hold:

$\phi_d$ is from 80° to 100°, typically from 86° to 94°, more typically from 87.6° to 92.6°;

$\phi_p$ is from 76° to 96°, typically from 82° to 92°, more typically from 83.5° to 89.9°; and

$\phi_d - \phi_p$ is from -5° to 10°, typically from -3.0° to 6.2°, more typically from 1° to 5°, such as 3.5°.

Fig. 2c) further illustrates the effects of an axial rotation. Starting from the neutral position shown, in which the proximal and distal parts contact only in the respective main saddle points, a small axial rotation around the z-axis is substantially unhindered. Therein, "substantially unhindered" means unhindered apart e.g. from frictional forces, or at least not hindered by the compressive force acting in the z-direction. Under such a small axial rotation, the contact point, corresponding to the respective saddle points, does not change.

But, when one of the straight lines of the proximal surface meets a respective straight line of the distal surface, the contact point is enlarged to a contact line. When the axial rotation is further increased, the contact line splits into two new contacts points different from the original contact point at the respective main saddle points of the articulating surfaces. The two new contact points are located at outer areas of the articulating surfaces. The main saddle points of the respective articulating saddle shaped surfaces (which formed the former contact point) lift off. Thus the two artificial bones (the artificial trapezium and the artificial metacarpal bone) spiral apart and produce a dehiscence in the center of the joint. Compressive joint forces in the two contacts, which can be produced by muscles and/or ligaments, counteract this dehiscence by producing an increasing counteracting torque. This torque handicaps or hinders further axial rotation, and spirals the joint back. As a result, an artificial CMCJ according to embodiments described herein is adapted to stabilize the axial rotation in a simple way: The muscles only need to produce a compressive resultant force. When twisted in a way that a contact line or the two contact points with resulting counter-torque result, the mechanical joint can lose one effective degree of freedom. That means, for any given torque strength applied to the joint, the range of axial rotation is limited. The kinematical degrees of freedom describing the motion need not be reduced. For example, if starting out with five kinematical degrees of freedom (e.g. point contact in the main saddle points), the effective degrees of freedom are reduced to four in certain second position corresponding to an angle of relative axial rotation larger than the maximal angle of axial rotation, wherein the second position is such that the counter-torque resulting from the compressive force balances the torque applied to the joint.

For the mechanical joint illustrated in Fig. 2c), the maximal angle of substantially free axial rotation is $\pm| \phi_p - \phi_d |/2$. If starting from a non-symmetric neutral position, e.g. with a pre-tilt of $\pm| \phi_p - \phi_d |/2$, then the maximal angle of axial rotation is $| \phi_p - \phi_d |$ at most. For the case $\phi_p - \phi_d = 0$, both pairs of straight lines $p_1$, $d_1$ and $p_2$, $d_1$, respectively, coincide. The saddle-shaped regions contact each other in two line contacts, and the axial rotation is handicapped for every rotation angle different from zero. In this case, first and second parts of the mechanical joint form, in the neutral position of the mechanical joint, an articulated connection under the compressive force in two contact lines of the first and second curved surfaces. This case shall be included when specifying that first and second parts form a connection in a contact point.

Generally, according to some embodiments, the angular difference $|\phi_d - \phi_p|$ may be equal to a maximal angle of relative axial rotation. The maximal angle of relative axial rotation is the rotation angle beyond which, i.e. for all angles larger than the maximal angle, at least one of the following holds: (i) the relative axial rotation is handicapped or hindered, (ii) an axial compressive force produces a counter-torque, (iii) there are at least two contact points of the curved surfaces, (iv) the contact point for angles smaller than the maximal angle on the respective surfaces, typically the main saddle points of the saddle-shaped regions, is/are lifted off. The angles of relative axial rotation are denoted with non-negative values irrespective of clockwise or anti-clockwise twisting.

The mechanical joint can be a self-stabilizing joint. A self-stabilizing joint means a joint resisting, solely by means of a compressive force, to clockwise and anti-clockwise axial rotation for rotation angles larger in magnitude than the maximal angle of relative axial rotation. The mechanical joint may be called a left-right self-torquing-down joint. As a vague comparison, not meant as a limitation in any way, one may imagine a screw pulled down and tightened when turned in either direction.

According to an embodiment, a mechanical joint, e.g. an endoprosthesis for a carpometacarpal joint, is provided. The mechanical joint includes a first part of the joint and a second part of the joint. The first part of the joint includes a first curved surface, and the second part of the joint includes a second curved surface. The first and second parts are adapted to form, in a first position of the mechanical joint, an articulated connection in a contact point under a compressive force, e.g. a compressive axial force. The first and second curved surfaces are shaped such that, for a second position of the mechanical joint corresponding to a relative rotation angle larger than a maximal angle of relative rotation, the contact point of the first and of the second curved surfaces lifts off. The first and second curved surfaces may include respective regions that include the common contact point, and in which regions the first surface and/or second surface are saddle-shaped. However, differently shaped curved surfaces might be conceived.

The first and second curved surfaces may generally be adapted to handicap a relative axial rotation of the first part relative to the second part under a compressive force. Therein, the relative axial rotation is handicapped for relative rotation angles larger than, or equal to, a maximal angle of (substantially unhindered) relative axial rotation.

Generally, the maximal angle of relative axial rotation can e.g. lie in the range from 0° to 10°, more typically from 1.5° to 5.5°, even more typically from 3° to 4°, such as 3.5°. The mechanical joint may have five kinematical degrees of freedom in the first position, typically the neutral position. The mechanical joint may have less than five effective degrees of freedom in a second position, wherein the second position of the joint corresponds to a relative axial rotation of the first and second part of the mechanical joint under the compressive force with a relative rotation angle larger than the maximal angle of relative axial rotation.

According to some embodiments, at least one of the following holds for the principal radii of the articulating curved surfaces: $R_{ap}$ may be in the range from 8 mm to 19 mm, $R_{fp}$ may be in the range from -4 mm to -11 mm, $R_{ad}$ may be in the range from 8 mm to 14 mm, $R_{fp}$ may be in the range from -5 mm to -10 mm. The minus sign denotes that the corresponding center of curvature is on the proximal side (z-coordinate < 0) as shown in Figs. 2a) and 2b). Further, in some embodiments, at least one of the following may hold: $(R_{ap}-R_{ad})$ may be in the range from 1.4 to 3.4 mm; and $(R_{fp}-R_{fd})$ may be in the range from 0.4 to 1.2 mm.

The compressive force may be exerted by muscles. These may also guide the motion of the mechanical joint. In guiding the joint motion the two articulating surfaces and their curvatures should be considered. In other words, the two articulating surfaces of a diarthrose should have biomechanically equal rights in a biomechanical consideration. Hence out of neutral position, initial rotations can be performed around the morphological defined center of curvature $M_{fd}$ or $M_{fp}$ or simultaneously around both (flexion/extension, Fig. 2a), or around the two centers $M_{ad}$, and $M_{ap}$, of ab-/adduction as shown in Fig. 2b). These 4 kinematical degrees of freedom (4 DOF) are controlled by eight muscles (4 muscles for flexion/extension and 4 muscles for ab-/adduction). Out of neutral position, a small axial rotation is at first possible without that the contact at the saddle points is changed (Fig. 2c), since the angles between the straight lines of the saddle joints do not coincide. Therefore, the CMCJ initially has five DOFs. As explained above, the axial rotation in the CMCJ can be effectively stabilized by the muscular system, in particular by the compressive forces of the 8 muscles, reducing the DOFs to 4 outside of a range of small axial rotations.

Circumduction of the mechanical joint can still be performed by the musculature. In vivo the musculature is able to guide the extended thumb along a cone whose apex seems to lie in the CMCJ. In the course of this motion the metacarpal bone does not rotate around its long axis (no axial rotation). For that ab-/adduction und flexion/extension have to occur simultaneously in an alternating manner. Because ab-/adductions can be produced by rotations around two independent axes ($M_{ad}$, $M_{ap}$), it is possible that the resulting instantaneous axis of ab-/adduction ($IRA_a$) runs through the common saddle point O (Fig. 3a). The same procedure is possible in flexion (instantaneous axis: $IRA_f$). In both cases the angular velocities $\omega_{ij}$ of the respective rotations must then show distinct relations which are described by the following formulae:

$$-\frac{\omega_{ap}}{\omega_{ad}} = \frac{R_{ad}}{R_{ap}} \quad \text{and} \quad -\frac{\omega_{fp}}{\omega_{fd}} = \frac{R_{fd}}{R_{fp}}$$

The two instantaneous axes of ab-/adduction and flexion/extension compose then to a resulting instantaneous axis $IRA_{res}$ (Fig. 3b). The instantaneous motion is given by vector addition of the vectors angular velocities:

$$\underline{\omega}_{res} = \underline{\omega}_a - \underline{\omega}_f = \omega_a \cdot \underline{e}_y - \omega_f \cdot \underline{e}_x$$

When the 8 muscles operate in such a way that they accelerate the metacarpal bone always perpendicularly to the vector of the resulting angular velocity $\omega_{es}$, the resulting instantaneous axis $IRA_{res}$ pivots around the saddle point O. The articulating surfaces move in a rolling manner. In the simplest case the angular velocities of ab-/adduction $\omega_a$ and of flexion/extension $\omega_f$ alternate in phase-delayed harmonic time functions. Then the long axis of the metacarpal bone moves on a cone-shaped shell without that the bone carries out a rotation around its long axis. Precondition for this mechanism is that the 8 muscles independently alter tonus and length what is physiologically possible. Since the musculature, which stimulates the circumduction, produces a compressive force, axial rotation is stabilized and does hardly appear together with circumduction.

While the foregoing is directed to embodiments of the invention, other and further embodiments of the invention may be devised without departing from the basic scope thereof, and the scope thereof is determined by the claims that follow.

**Claims**

1. A mechanical joint, in particular endoprosthesis, for example for a carpometacarpal joint, comprising:

     - a first part of the joint, including a first curved surface; and
     - a second part of the joint, including a second curved surface,
     the first and second parts being adapted to form, in a first position of the mechanical joint, an articulated connection under a compressive force in a contact point of the first and second curved surfaces,
wherein the first and second curved surfaces are saddle-shaped at least in a region including the contact point.

2. The mechanical joint according to claim 1, wherein the contact point in the first position of the mechanical joint under the compressive force is a main saddle point of the first curved surface and/or of the second curved surface.

3. The mechanical joint according to any of the preceding claims, wherein the first and second curved surfaces are incongruent at the contact point in one or both principal directions of curvature.

4. The mechanical joint according to any of the preceding claims, wherein the principal radii of curvature of the saddle-shaped region of the first curved surface at the contact point are $R_{ap}$ and $R_{fp}$, and the principal radii of curvature of the saddle-shaped region of the second curved surface at the contact point are $R_{ad}$ and $R_{fd}$, and wherein one or both of the following relations hold:

$$|R_{ap}| \neq |R_{ad}| \text{ and } |R_{fp}| \neq |R_{fd}|,$$

wherein |●| denotes the absolute value.

5. The mechanical joint according to claim 4, wherein one or both of the following relations hold: $|R_{ap}| > |R_{ad}|$ and $|R_{fp}| < |R_{fd}|$.

6. The mechanical joint according to any of the preceding claims, wherein there is a maximal angle of relative rotation, and wherein the first and second curved surfaces are shaped such that, for a second position of the mechanical joint corresponding to a relative rotation angle larger than the maximal angle of relative rotation, the contact point of the first and of the second curved surfaces lifts off.

7. The mechanical joint according to any of the preceding claims, wherein the first and second curved surfaces are adapted to handicap a relative axial rotation of the first part relative to the second part under the compressive force for relative rotation angles larger than, or equal to, a maximal angle of relative axial rotation.

8. The mechanical joint according to any of the preceding claims, wherein the saddle-shaped region of the first curved surface and/or the saddle shaped region of the second curved surface are saddle surfaces of higher than second order.

9. The mechanical joint according to any of the preceding claims, wherein the intersections of the first saddle-shaped region with two surface normal planes at the contact point of the first saddle-shaped region form two straight intersection lines $p_1$ and $p_2$, and the intersections of the second saddle-shaped region with two surface normal planes at the contact point of the second saddle-shaped region form two straight intersection lines $d_1$ and $d_2$,
wherein $p_1$ and $p_2$ form an angle $\phi_p$ in a first sector, and $d_1$ and $d_2$ form an angle $\phi_d$ in a second sector, the first and second sector overlapping by more than 45°, and at least one, at least two, or all of the following relations hold:

$\phi_d$ is from 80° to 100°, typically from 86° to 94°, more typically from 87.6° to 92.6°;
$\phi_p$ is from 76° to 96°, typically from 82° to 92°, more typically from 83.5° to 89.9°; and
$\phi_d$ - $\phi_p$ is from -5° to 10°, typically from -3.0° to 6.2°, more typically from 1° to 5°, such as 3.5°.

10. The mechanical joint according to the preceding claim, wherein the angular difference $\phi_d$ - $\phi_p$ is equal to the maximal angle of relative axial rotation.

11. The mechanical joint according to any of the preceding claims, wherein the maximal angle of relative axial rotation lies in the range from 0° to 10°, more typically from 1.5° to 5.5°, even more typically from 3° to 4°, such as 3.5°.

12. The mechanical joint according to any of the preceding claims, wherein the joint has five kinematical degrees of freedom in the first position.

13. The mechanical joint according to any of the preceding claims, wherein the joint has less than five effective degrees of freedom in a second position, wherein the second position of the joint corresponds to a relative axial rotation of the first and second part of the mechanical joint under the compressive force with a relative rotation angle larger than the maximal angle of relative axial rotation.

**Fig. 1**

**Fig. 2**

**Fig. 3**

Europäisches
Patentamt

European
Patent Office

Office européen
des brevets

# EUROPEAN SEARCH REPORT

Application Number

EP 10 00 2528

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 95/09587 A1 (ORTHOMET INC [US]; MAYO FOUNDATION [US]) 13 April 1995 (1995-04-13) * page 1, line 16 - page 9, line 24; figures 2-9 * | 1-13 | INV. A61F2/42 ADD. A61F2/00 |
| X | EP 0 669 117 A1 (SUTTER CORP [US]) 30 August 1995 (1995-08-30) * column 4, line 48 - column 16, line 40; figures 2,5,6,7,12 * | 1-13 | |
| X | EP 0 455 929 A1 (THERA GES FUER PATENTE [DE]) 13 November 1991 (1991-11-13) * column 1, line 38 - column 5, line 38; figures 1-6 * | 1-13 | |
| X | FR 2 805 151 A1 (CREMASCOLLI ORTHO S A [FR]) 24 August 2001 (2001-08-24) * page 2, line 8 - page 5, line 23; figure 1 * | 1-13 | |
| X | US 2002/065561 A1 (OGILVIE WILLIAM F [US] ET AL) 30 May 2002 (2002-05-30) * paragraph [0016] - paragraph [0068]; figures 3-8 * | 1-13 | TECHNICAL FIELDS SEARCHED (IPC) A61F |

The present search report has been drawn up for all claims

| Place of search | Date of completion of the search | Examiner |
|---|---|---|
| Munich | 13 August 2010 | Cuiper, Ralf |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 10 00 2528

This annex lists the patent family members relating to the patent documents cited in the above-mentioned European search report.
The members are as contained in the European Patent Office EDP file on
The European Patent Office is in no way liable for these particulars which are merely given for the purpose of information.

13-08-2010

| Patent document cited in search report | | Publication date | Patent family member(s) | | Publication date |
|---|---|---|---|---|---|
| WO 9509587 | A1 | 13-04-1995 | AU | 680559 B2 | 31-07-1997 |
| | | | AU | 7927794 A | 01-05-1995 |
| | | | CA | 2173581 A1 | 13-04-1995 |
| | | | EP | 0726746 A1 | 21-08-1996 |
| | | | JP | 9506009 T | 17-06-1997 |
| | | | US | 5405400 A | 11-04-1995 |
| EP 0669117 | A1 | 30-08-1995 | EP | 0671153 A1 | 13-09-1995 |
| | | | JP | 7241306 A | 19-09-1995 |
| | | | JP | 7194632 A | 01-08-1995 |
| | | | US | 5522900 A | 04-06-1996 |
| EP 0455929 | A1 | 13-11-1991 | AT | 121926 T | 15-05-1995 |
| | | | CA | 2041952 A1 | 12-11-1991 |
| | | | DE | 9005372 U1 | 26-09-1991 |
| | | | ES | 2071839 T3 | 01-07-1995 |
| | | | JP | 4231044 A | 19-08-1992 |
| FR 2805151 | A1 | 24-08-2001 | FR | 2805152 A1 | 24-08-2001 |
| US 2002065561 | A1 | 30-05-2002 | NONE | | |

EPO FORM P0459

For more details about this annex : see Official Journal of the European Patent Office, No. 12/82